# DEMANDE DE BREVET EUROPEEN

(11) **EP 0 992 253 A1**
(43) Date de publication de la demande: **12.04.2000**
(21) Numéro de dépôt: 98811015.1
(22) Date de dépôt: 09.10.1998
(51) Int. Cl.: A61M 1/08

(54) **Extracteur de venin**

(71) Demandeur: Rochat, Jean-Denis, 1272 Genolier (CH)
(72) Inventeur: Rochat, Jean-Denis, 1272 Genolier (CH)
(74) Mandataire: Moinas, Michel

(57) **Abrégé**

Extracteur de venin (2) actionnable par pression manuelle directe, destiné à être placé sur la peau (6) et à appliquer à ce niveau une pression négative par l'intermédiaire d'une ouverture (5), caractérisé en ce qu'il est constitué d'une structure en matière souple, élastique et étanche formant une cavité (1) élastiquement déformable, en ce que la pression négative est produite, après compression de ladite structure afin d'en vider l'air, par le relâchement élastique de celle-ci après application sur la peau, et en ce qu'il reprend progressivement sa forme initiale à mesure qu'il aspire le venin hors de la peau, tout en restant appliqué à la peau par l'effet de la pression négative régnant dans ladite cavité et de ses bords formant avec la peau un joint essentiellement étanche.

## Description

La présente invention concerne un dispositif pour extraire le venin des piqûres ou morsures d'insectes volants (par exemple guêpes, abeilles et frelons), rampants (par exemple serpents, scorpions) et autres animaux (par exemple poissons, coquillages) par l'application de vide au niveau de la peau permettant d'aspirer le venin.

On connaît diverses formes d'extracteurs à venin, généralement constitués d'une pompe à vide actionnée par un piston coulissant dans un cylindre appelé "corps de pompe" entre une position avancée et une position retirée. Au cours du déplacement du piston, ce dernier réalise un volume de vide égal à sa surface que multiplie sa course. De tels dispositifs sont par exemples décrits dans les documents FR 2 574 299, FR 2 554 002, FR 2 408 738, GB 2008200, WO 86/04819 et WO 97/14452, ainsi que dans la documentation commerciale décrivant le dispositif Aspivenin®.

Tous ces dispositifs son relativement sophistiqués et encombrants, comportent un nombre important de pièces et sont relativement coûteux à fabriquer. De plus, la plupart d'entre eux ne peuvent être actionnés que par l'usage des deux mains et leur manipulation peut être malaisée en cas de panique. La présente invention a pour objectif un dispositif simple, facile à manipuler, réutilisable et peu coûteux à fabriquer.

La présente invention concerne un extracteur de venin actionnable par pression manuelle directe, destiné à être placé sur la peau et à appliquer à ce niveau une pression négative par l'intermédiaire d'une ouverture, caractérisé en ce qu'il est constitué d'une structure en matière souple, élastique et étanche formant une cavité élastiquement déformable, en ce que la pression négative est produite, après compression de ladite structure afin d'en vider l'air, par le relâchement élastique de celle-ci après application sur la peau, et en ce qu'il reprend progressivement sa forme initiale à mesure qu'il aspire le venin hors de la peau, tout en restant appliqué à la peau par l'effet de la pression négative régnant dans ladite cavité et de ses bords formant avec la peau un joint essentiellement étanche.

En exploitant par pression manuelle directe les propriétés élastiques du matériau utilisé en lieu et place d'un système de piston compliqué pour créer une pression négative (comme dans l'Aspivenin® mentionné plus haut), la présente invention permet une production simplifiée comportant une seule pièce ou un nombre limité de pièces jointes, par exemple deux pièces soudées entre elles, d'un coût modique et d'un encombrement limité. En outre, le principe du dispositif est immédiatement compris par le public d'où un usage facile même dans des conditions de panique liées à une morsure ou piqûre d'animal. L'invention telle qu'exposée est en outre utilisable d'une seule main et les matériaux utilisés en permettent la réutilisation ultérieure.
La Figure 1a représente une coupe transversale d'une forme d'exécution d'un extracteur de venin selon l'invention.
La Figure 1b représente une vue en coupe transversale selon la ligne A-A de l'extracteur de venin représenté à la Figure 1a.
La Figure 1c représente une vue en coupe transversale selon la ligne A-A de la même forme d'exécution d'un extracteur selon l'invention, en train d'être comprimé avant application sur la peau.
La Figure 1d représente une vue en coupe trañsversale selon la ligne A-A de la même forme d'exécution d'un extracteur selon l'invention, en place sur la peau.
La Figure 1e représente une vue en coupe transversale selon la ligne B-B de la même forme d'exécution d'un extracteur selon l'invention, montrant des parois d'épaisseur variable.
La Figure 2 représente une coupe transversale d'une seconde forme d'exécution d'un extracteur selon l'invention.
La Figure 3 représente une coupe transversale d'une troisième forme d'exécution d'un extracteur selon l'invention, en train d'être comprimé.
La Figure 4 représente une coupe transversale d'une quatrième forme d'exécution d'un extracteur selon l'invention.
La Figure 5 représente une coupe transversale d'une cinquième forme d'exécution d'un extracteur selon l'invention, constitué d'une section de tuyau flexible dont les parois internes de l'une des extrémités sont jointes.

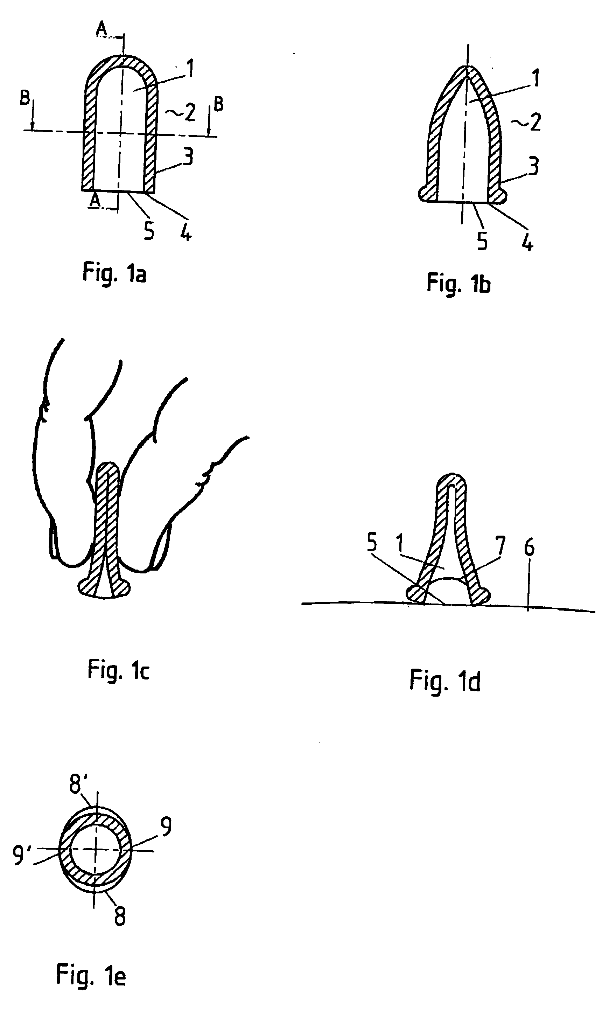

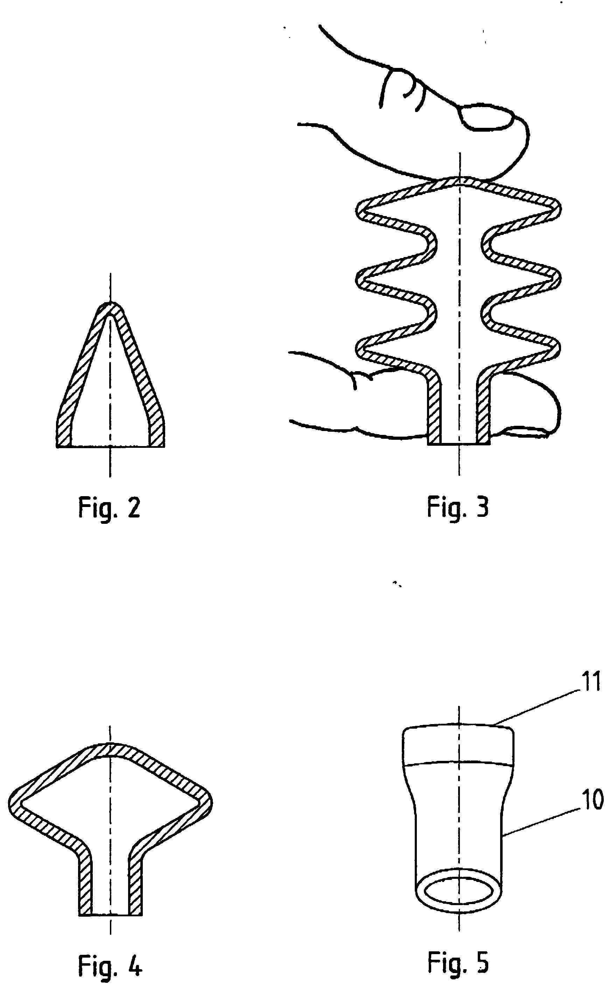

Les Figures 1a, 1b, 1c, et 1d montrent une forme d'exécution de l'invention respectivement en position de repos (1a, 1b), en train d'être comprimée avant placement sur la peau (1c), et après placement sur la peau et relâchement de la compression (1d). L'air contenu dans la cavité 1 est exprimé ou expulsé hors de l'extracteur à venin 2 par pression digitale sur les parois déformables. La paroi 3 de l'extracteur se termine autour de l'ouverture 5 par une lèvre 4, dont l'angle interne est typiquement vif, à coupe franche, droite ou en biseau. Cette disposition permet d'assurer une bonne étanchéité, laquelle peut encore être améliorée par l'addition de graisse silicone. Si désiré, la lèvre 4 peut en outre contenir une rainure éventuellement remplie de graisse silicone. La figure 1d montre l'extracteur en place sur la peau 6, transmettant à celle-ci la pression négative régnant dans la cavité 1 par l'intermédiaire de l'ouverture 5. On remarque que sous la pression négative, la peau forme un bourrelet 7 à travers l'ouverture 5. La surface intérieure de l'extracteur sera de préférence lisse à ce niveau, afin d'assurer une étanchéité optimale avec le bourrelet cutané 7. l'étanchéité peut être encore améliorée en humectant préalablement la peau par de l'eau ou de la salive, notamment dans les zones poilues. Typiquement, le diamètre intérieur du dispositif est compris entre 7 et 25 mm, l'épaisseur de sa paroi entre 2 et 5 mm, et sa hauteur totale entre 20 et 40 mm. De préférence cylindrique (Figures 1 et 5), l'extracteur à venin peut également avoir d'autres formes, par exemple conique (Figure 2), en accordéon (Figure 3) ou en poire (Figure 4). Il convient de relever que la cavité 1 formée par l'extracteur peut être adaptée selon le type d'animal dont on veut traiter la piqûre ou la morsure, ou selon l'endroit du corps où l'on veut aspirer.

Une forme d'exécution préférée d'un extracteur de venin selon l'invention, représentée à la Figure 5, est constituée d'une section de tuyau flexible 10 dont les parois internes de l'une des extrémités 11 sont soudées ensembles, collées ou jointes de toute autre manière. Cette forme d'exécution permer de faciliter sensiblement la fabrication de l'extracteur et de diminuer son coût.

La capacité de l'extracteur à venin à former une pression négative suffisante dépend de plusieurs facteurs, dont la nature et la dureté du matériau souple utilisé, la forme et le volume du dispositif ainsi que le rapport entre la surface de l'ouverture et le volume de la cavité. De préférence, la pression négative obtenue lors du relâchement élastique de l'extracteur appliqué à la peau est comprise entre -400 et -900 mBar.

De préférence également, le matériau utilisé sera du silicone, du PVC plastifié ou du caoutchouc. Sa dureté sera comprise entre 45° Shore A et 90° Shore A. Quant à la forme du dispositif, elle doit permettre à l'opérateur de le saisir et le comprimer aisément entre ses doigts de manière à placer son ouverture à la peau. Si désiré, l'épaisseur des parois de l'extracteur peut être variable entre elles afin d'assurer une rigidité et une efficacité optimale du dispositif. Par exemple, l'épaisseur des parois antérieure 8 et postérieure 8' d'un extracteur de forme cylindrique tel qu'illustré à la Figure 1e peut être plus importante que l'épaisseur de ses parois latérales 9 et 9', ce qui confère à l'extracteur une plus grande rigidité dans une axe et une plus grande flexibilité dans un autre. Si la peau est poilue, il est recommandé de mouiller ou humecter la lèvre 4 avant d'appliquer l'extracteur de venin. Le volume compris à l'intérieur de l'extracteur pendant compression manuelle et le volume compris à l'intérieur de l'extracteur après application sur la peau et relâchement de la compression manuelle sont avantageusement dans un rapport de 2,5 à 4,5. Selon la dureté du matériau utilisé, le rapport volume pendant/après compression manuelle ne devra pas tomber au-dessous d'un niveau de l'ordre de 1,5. Par exemple, un dispositif de type ventouse en caoutchouc ou coupelle ne forme pas un volume suffisant et a un rapport volume pendant/après compression manuelle trop petit pour être utilisé dans l'application décrite ici. De plus, un tel dispositif nécessite d'exercer une pression directement sur le point de piqûre ou morsure, ce qui peut être douloureux.

## Revendications

1. Extracteur de venin (2) actionnable par pression manuelle directe, destiné à être placé sur la peau (6) et à appliquer à ce niveau une pression négative par l'intermédiaire d'une ouverture (5), caractérisé en ce qu'il est constitué d'une structure en matière souple, élastique et étanche formant une cavité (1) élastiquement déformable, en ce que la pression négative est produite, après compression de ladite structure afin d'en vider l'air, par le relâchement élastique de celle-ci après application sur la peau, et en ce qu'il reprend progressivement sa forme initiale à mesure qu'il aspire le venin hors de la peau, tout en restant appliqué à la peau par l'effet de la pression négative régnant dans ladite cavité et de ses bords formant avec la peau un joint essentiellement étanche.

2. Extracteur de venin selon la revendication 1, caractérisé en ce que la dépression produite dans ladite cavité (1) lors du relâchement élastique de l'extracteur appliqué à la peau (6) est comprise entre -400 et -900 mBar.

3. Extracteur de venin selon l'une des revendications 1 et 2, caractérisé en ce que ladite matière souple a une dureté comprise entre 45° Shore A et 90° Shore A..

4. Extracteur de venin selon l'une des revendications 1 à 3, caractérisé en ce que son diamètre intérieur est compris entre 7 et 25 mm, l'épaisseur de sa paroi (3) entre 2 et 5 mm, et sa hauteur totale entre 20 et 40 mm.

5. Extracteur de venin selon l'une des revendications 1 à 4, caractérisé en ce que ladite ouverture (5) d'application à la peau comporte une lèvre (4) à angle interne vif.

6. Extracteur de venin selon l'une des revendications 1 à 5, caractérisé en ce que le volume compris à l'intérieur de l'extracteur pendant compression manuelle et le volume compris à l'intérieur de l'extracteur après application sur la peau et relachement de la compression manuelle sont dans un rapport de 2,5 à 4,5.

7. Extracteur de venin selon l'une des revendications 1 à 6, caractérisé en ce que la matière souple est du silicone.

8. Extracteur de venin selon l'une des revendications 1 à 6, caractérisé en ce que la matière souple est du PVC plastifié.

9. Extracteur de venin selon l'une des revendications 1 à 6, caractérisé en ce que la matière souple est du caoutchouc.

10. Extracteur de venin selon l'une des revendications précédentes, caractérisé en ce qu'il est constitué d'une section de tuyau flexible (10) dont les parois internes de l'une des extrémités (11) sont jointes.

11. Extracteur de venin selon l'une des revendications précédentes, caractérisé en ce qu'il est réutilisable après usage initial.

12. Extracteur de venin selon l'une des revendications précédentes, caractérisé en ce que ses parois (8, 8', 9, 9') ont une épaisseur variable entre elles de manière à lui conférer une rigidité variable dans les différents axes.
